# EUROPÄISCHE PATENTSCHRIFT

(11) **EP 1 012 246 B1**
(45) Veröffentlichungstag und Bekanntmachung des Hinweises auf die Patenterteilung: **24.05.2006**
(21) Anmeldenummer: 98951376.7
(22) Anmeldetag: 10.09.1998
(51) Int. Cl.: C12N 1/10

(54) **FERMENTATIONSVERFAHREN MIT KONTINUIERLICHER MASSENKULTIVIERUNG VON CILIATEN (PROTOZOA) ZUR PRODUKTION BIOGENER WERTSTOFFE**
FERMENTATION METHOD WITH CONTINUOUS MASS CULTIVATION OF CILIATES (PROTOZOA) FOR PRODUCING BIOGENOUS VALUABLE SUBSTANCES
PROCEDE DE FERMENTATION AVEC MISE EN CULTURE DE MASSE CONTINUE DE CILIES (PROTOZOAIRES) POUR LA PRODUCTION DE SUBSTANCES BIOGENES VALORISABLES

(30) Priorität: 19.09.1997 DE 19741489
(43) Veröffentlichungstag der Anmeldung: 28.06.2000
(73) Patentinhaber: Nutrinova Nutrition Specialties & Food Ingredients GmbH, 65926 Frankfurt am Main (DE)
(72) Erfinder: KIY, Thomas, D-65929 Frankfurt (DE)
(74) Vertreter: Luderschmidt, Schüler & Partner GbR
(86) Internationale Anmeldenummer: PCT/EP1998/005770
(87) Internationale Veröffentlichungsnummer: WO 1999/015634

(56) Entgegenhaltungen:
- DE-A- 4 238 842
- FR-A- 2 334 630
- CHEMICAL ABSTRACTS, vol. 66, no. 5, 30. Januar 1967 Columbus, Ohio, US; abstract no. 17297, ROSENBAUM, NANCY ET AL: "Induction of a phospholipid requirement and morphological abnormalities in Tetrahymena pyriformis by growth at supraoptimal temperatures" XP002090950 & J. PROTOZOOL. (1966), 13(4), 535-46 CODEN: JPROAR,
- CHEMICAL ABSTRACTS, vol. 94, no. 9, 2. März 1981 Columbus, Ohio, US; abstract no. 61384, YAMIN, MICHAEL A.: "Cellulose metabolism by the flagellate Trichonympha from a termite is independent of endosymbiotic bacteria" XP002090951 & SCIENCE (WASHINGTON, D. C., 1883-) (1981), 211(4477), 58-9 CODEN: SCIEAS;ISSN: 0036-8075,
- WILLIAM TRAGER ET AL.: "Human malaria parasites in continuous culture" SCIENCE, Bd. 193, 20. August 1976, Seiten 673-675, XP002090949 US
- CHEMICAL ABSTRACTS, vol. 76, no. 9, 28. Februar 1972 Columbus, Ohio, US; abstract no. 43778, VAN WAGTENDONK, W. J. ET AL: "Axenic cultivation of Paramecium aurelia" XP002090952 & METHODS CELL PHYSIOL. (1970), 4, 117-30 CODEN: MCPHA6,
- CHEMICAL ABSTRACTS, vol. 70, no. 21, 26. Mai 1969 Columbus, Ohio, US; abstract no. 94181, CURDS, C. R. ET AL: "Growth and feeding of Tetrahymena pyriformis in axenic and monoxenic culture" XP002090953 & J. GEN. MICROBIOL. (1969), 54(3), 343-58 CODEN: JGMIAN,1969,

## Beschreibung

Die Erfindung betrifft ein Fermentationsverfahren mit kontinuierlicher Massenkultivierung von Ciliaten (Protozoa) zur Produktion biogener Wertstoffe, bei dem die die biogenen Wertstoffe enthaltende Biomasse durch kontinuierlichen (permanenten) Zellaustrag gewonnen werden.

Die biotechnologische Nutzung von Ciliaten - einer Klasse der Protozoen - ist bisher nur ansatzweise realisiert, obwohl zahlreiche Stoffwechselprodukte dieser Organismen von wirtschaftlichem Interesse sind, z.B lysosomale Enzyme. Derzeit sind nur wenige biotechnologische Verfahren zur Gewinnung von biogenen Wertstoffen aus Ciliaten beschrieben, - vorwiegend für das Ciliat Tetrahymena (Kiy & Tiedtke, 1991, Appl. Microbiol. Biotechnol., 35, 14; Kiy et al., 1996, Enzyme Microb. Technol.. 18, 268; Kiy & Tiedtke, 1992, Appl. Microbiol. Biotechnol., 38, 141 ), - und es handelt sich hierbei ausschließlich um Verfahren zur Gewinnung von ausgeschiedenen Zeliprodukten, d.h. von solchen Zellprodukten, die von den Ciliatenzellen ins Kulturmedium abgegeben werden. Bei dieser Art von Verfahren werden die Ciliaten in Fermentern kultiviert und das die ausgeschiedenen biogenen Wertstoffe enthaltende Kulturmedium wird periodisch, in mehr oder weniger regelmäßigen Zeitabständen, abgenommen und gegen frisches Medium ausgetauscht. Während des Mediumaustauschs werden die Ciliaten über bestimmte Verfahren - z.B. den Einsatz von Membranen, eine Zellimmobilisierung o.ä. - im Fermenter zurückgehalten, so daß praktisch kein Zellmaterial verloren geht und die Zellkultur im Prinzip permanent fortbesteht.
Zur Gewinnung von biogenen Wertstoffen, die zellgebunden vorliegen, ist es jedoch nötig, die gesamten Zellen, die sog. Biomasse, zu ernten. Zu diesem Zweck wird in der Regel - d.h. bei den allgemein bekannten Fermentationsverfahren mit Bakterien oder Pilzen als Wertstoffproduzenten - eine sog. Batch-Fermentation durchgeführt, bei der der Fermenter angeimpft wird und die Zellen solange kultiviert werden, bis die maximale Biomasse bzw. Produktkonzentration erreicht ist. Dann wird die Biomasse geerntet. Derartige Verfahren sind auch bereits für verschiedene Ciliaten wie Paramecium, Colpoda und Tetrahymena beschrieben worden (Proper & Garver, 1966, Biotechnol. Bioeng., 8, 287, Schönefeld et al., 1986, J. Protozool., 33, 222: Kiy & Tiedtke, 1992, Appl. Microbiol. Biotechnol., 37, 576).

Die Batch-Fermentations-Verfahren haben jedoch den grundsätzlichen Nachteil, daß sie ein intervallmäßiges Reinigen, neues Animpfen des Fermenters und eine intensive Überwachung und Pflege der Zellkultur - vor allem während der kritischen Anwachsphase - erfordern.

Aus der Fermentationstechnik mit Bakterien oder Hefen als Wertstoffproduzenten ist neben dem Batch-Fermentationsverfahren auch das "kontinuierliche Fermentationsverfahren" bekannt. Bei diesem Verfahren werden die Zellen im Fermenter bis zu einer bestimmten Zelldichte gezüchtet und dann ständig durch kontinuierlichen Zellaustrag aus dem Fermenter geerntet während gleichzeitig im selben Umfang frisches Kulturmedium zugeführt wird. Die pro Zeiteinheit entnommene Zellmenge (der Zellaustrag) ist so bemessen, daß die im Fermenter verbleibenden Zellen die durch die Ernte bedingte Abnahme der Zelldichte durch kontinuierliche Zellteilungen mühelos wieder ausgleichen können. Im Bereich einer bestimmten Zellaustragsrate bzw. Verdünnungsrate "D" bleibt die Zelldichte im Fermenter somit konstant, obwohl kontinuierlich Kultur und damit das gewünschte Produkt geerntet wird.

Prinzipiell ist dieses kontinuierliche Fermentationsverfahren einer Batch-Fermentation ökonomisch weit überlegen, aber seine Durchführung setzt voraus, daß die kultivierten bzw. gezüchteten Organismen relativ schnell und gleichmäßig wachsen und sich vermehren, und daß sie unempfindlich gegen die Rühr- und Scherkräfte sind, die bei einem kontinuierlichen Fermentationsverfahren auftreten.

Von Ciliaten ist hingegen allgemein bekannt, daß sie häufig nur sehr langsam wachsen und sich vermehren, daß sie unterschiedliche Wachstumsphasen durchlaufen, und daß sie sehr empfindlich auf Rühr- und Scherkräfte reagieren (Curds & Cockburn. 1971, Journal of General Microbiology 66. 95-109; Middler & Finn, 1966, Biotechnology and Bioengineering 8, 71-84). Zwar sind auch schon Versuche zur kontinuierlichen Massenkultivierung von Ciliaten beschrieben worden, aber ausschließlich unter Verwendung von bakterienhaltigen Kulturmedien und mit Ergebnissen zur maximalen Zelldichte von wenigen Zehntausend Zellen pro ml trotz 10 Tagen Kultivierung und länger (Curds & Cockbum, supra).
Solche Zelldichten sind für einen Einsatz im großtechnischen, industriellen Maßstab völlig unzureichend. Darüberhinaus ist die von Curds & Cockburn beschriebene Kultivierung auch deshalb für einen großtechnischen Einsatz gänzlich ungeeignet, weil sie die Verwendung von beuteorganismen-, nämlich bakterienhaltigem Medium vorschreibt. Bei einem bakterienhaftigen Kulturmedien kommt es selbstverständlich auch zu einer ständigen Weitervermehrung der Bakterien, und zwar im Umfang abhängig davon, wieviele Ciliaten vorhanden sind. Das Koexistenzgleichgewicht von Ciliatenpopulation und Bakterienpopulation ist sehr labil, und schon ein geringfügiger Eingriff kann gravierende Veränderungen bei beiden Populationen hervorrufen.

Die Versuche von Curds & Cockburn liegen überdies mehr als 25 Jahre zurück und haben die Fachwelt offensichtlich in ihrer Meinung bestärkt, daß Ciliaten für ein kontinuierliches Fermentationsverfahren im großtechnischen Maßstab nicht geeignet sind.

Der vorliegenden Erfindung liegt nun die Aufgabe zugrunde, ein Verfahren zur kontinuierlichen Fermentation durch Ciliaten mit Zellaustrag bereitzustellen, bei dem die genannten Nachteile vermieden sind und das insbesondere für den großtechnischen, industriellen Einsatz gut geeignet ist.

Eine Lösung dieser Aufgabe besteht in der Bereitstellung eines Verfahrens der Eingangs genannten Art, bei dem die Ciliatenzellen in komplexem, axenischem Medium kultiviert werden.

Im Sinne dieser Erfindung bedeutet komplexes Medium ein Nährmedium wäßriger Lösung von Naturprodukten bzw. aus diesen gewonnenen Extrakten zur Kultivierung von Mikroorganismen.

Im Sinne dieser Erfindung bedeutet axenisches Medium ein, von Futter- und Beuteorganismen (sogenannte Nahrungsorganismen) freies Nährmedium.

Das erfindungsgemäße Verfahren beruht auf der überraschenden Erkenntnis, daß ein kontinuierliches Fermentationsverfahren mit Zellaustrag unter Verwendung komplexer axenischer Medien auch mit reinen Ciliatenkulturen erfolgreich und wirtschaftlich außerordentlich rentabel durchführbar ist. Kontinuierliche Zelldichten in der Größenordnung von 1 Millionen Zellen pro ml sind ohne weiteres realisierbar, im Fall von Tetrahymena bereits ab dem dritten Tag nach Beginn der Kultivierung. Damit ist das Vorurteil der Fachwelt überwunden, daß Ciliaten für eine kontinuierliche Massenkultivierung mit Zelldichten von mehreren Hunderttausend bis Millionen Zellen pro ml unter Einsatz von bekannten Fermentern und in Gegenwart der üblicherweise zusammen auftretenden Scherkräfte, in axenischem Medium - d.h. ohne lebende Futter- bzw. Beuteorganismen - nicht geeignet sind, weil:
- sie zu langsam und zu ungleichmäßig wachsen,
- sie nur geringe Widerstandskräfte gegen Rühr- und Scherkräfte haben und sehr leicht und schnell durch solche Kräfte geschädigt bzw. zerstört werden, und
- bei den bisherigen Kultivierungsversuchen trotz Verwendung von Beuteorganismenhaltigem Medium und damit weitgehend naturgetreuem Nahrungsangebot nur verhältnismäßig sehr geringe maximale Zelldichten erreicht wurden.

Mit dem erfindungsgemäßen Verfahren ist es erstmals möglich, Ciliaten zur großtechnischen Produktion von zellgebundenen biogenen Wertstoffen einzusetzen und damit insbesondere solche Wertstoffe, die nur von Ciliaten bekannt sind, wie z.B. Taurolipide und Tetrahymanol, oder die speziell von Ciliaten in großem Umfang gebildet werden, wie z.B. Gamma-Linolensäure, Docosahexaensäure, Eicosapentaensäure, Octatetraensäure und Arachidonsäure, in wirtschaftlich bedeutendem Umfang zu gewinnen.

Da die Ciliaten als Reinkultur - d.h. frei von anderen lebenden Organismen - gehalten werden, sind wesentliche Störfaktoren von vorne herein vermieden, und auch der technische Aufwand ist auf ein Mindestmaß beschränkt: Fermenter zur Nachzucht der Beuteorganismen sind beispielsweise vollständig entfallen.

Zu den Produkten, die aus der ausgetragenen Biomasse gewonnen werden können, gehören Peptide und Proteine, vor allem Enzyme (z.B. β-Hexosaminidase, L-Asparaginase, Diisopropylfluorophosphatase, Glucosidase, Fucosidase, Phosphatase Nuklease oder Cathepsin.L), Fettsäuren und Lipide (z,B. Gamma-Linolensäure, Docosahexaensäure, Eicosapentaensäure, Octatetraensäure, Arachidonsäure, Phosphonolipide, Taurolipide, oder Tetrahymanol), Polysaccharide, Nukleinsäuren, Sekundärmetabolite, Polymerer, u.a..
Auch die Biomasse als solche kann das Produkt sein.

Die Gruppe der Ciliaten, die sich mittels des beschriebenen Verfahrens kultivieren lassen, umfaßt alle taxonomischen Ciliaten-Untergruppen, die sich prinzipiell in konventionellen Stand- und/oder Schüttelkulturen bzw. Batch-Fermentationen auf axenischen Nährmedien bzw. Nährmedien, die als Nährstoff abgetötete Biomasse eines Futterorganismus enthalten, kultivieren lassen. Dies sind insbesondere die Ciliatenunterklassen Holotricha, Peritricha, Spirotricha und Suctoria und ganz besonders die Gattungen Tetrahymena, Paramecium, Colpoda, Glaucoma, Parauronema, Engelmanniella, Stylonichia, Euplotes und Colpidium (Klassifizierung nach K. Hausmann: Protozoologie, Thieme Verlag, 1985). Die Erfindung ist auch nicht auf Wildstämme beschränkt, sondern schließt Mutanten und rekombinante Stämme ein.

In einer bevorzugten Ausführungsform des erfindungsgemäßen Verfahrens wird die Fermentation in einem Rühr-, oder Blasensäulen- oder Airliftfermenter durchgeführt.

Während der Fermentation kann der pH-Wert reguliert werden, vorzugsweise auf einen Wert im Bereich von pH 4 bis pH 9.
Die Fermentationstemperatur liegt je nach Ciliatenspezies zwischen 15 und 40°C.

Als Kohlenstoff-Quelle wird vorzugsweise wenigstens eine der nachfolgend aufgelisteten Substanzen verwendet, nämlich: Glucose, Fructose, Xylose, Saccharose, Maltose. Stärke, Fucose. Glucosamin, Lactose, Melasse, Dextran, Fettsäuren (z.B. Ölsäure), Sojaöl, Sonnenblumenöl, Glycerin, Glutaminsäure, Mannitol, Magermilchpulver oder Acetat.

Die Konzentration der Kohlenstoff-Quelle sollte zwischen 0,2 und 20 Gewichts-%, bezogen auf das Kulturmedium, liegen.

Als Stickstoff-Quelle wird vorzugsweise wenigstens eine der nachfolgend aufgelisteten Substanzen verwendet, nämlich: Peptone, Hefeextrakt, Malzextrakt, Fleischextrakt, Magermilchpulver, Casamino Acid, Corn Steep Liquor, organische Stickstoff-Quellen wie Na-Glutamat und Harnstoff, anorganische Stickstoff-Quellen wie Ammoniumacetat, Ammoniumsulfat, Ammoniumchlorid oder Ammoniumnitrat.
Die Konzentration der Stickstoff-Quelle sollte zwischen 0,1 und 10 Gewichts-%, bezogen auf das Kulturmedium, liegen.

Bei einer Variante des erfindungsgemäßen Verfahrens wird dem Kulturmedium wenigstens eine Phosphatquelle, z.B. Kaliumphosphat oder Kalium-Dihydrogenphosphat, zugesetzt. Alternativ oder kumulativ kann auch Ammoniumsulfat, Natriumsulfat, Magnesium, Eisen, Kupfer, Calcium, Vitamine, Spurenelemente und Wachstumsfaktoren zugesetzt werden, um die Wachstums- und Vermehrungsrate der betreffenden Ciliatenkultur weiter zu optimieren.

Die kontinuierlich geerntete Biomasse wird vom Kulturmedium vorzugsweise mittels Zentrifugation, Tangentialfiltration, Mikrofiltration, Sedimentation, Flotation oder Separatoren abgetrennt. Andere Methoden sind aber ebenfalls denkbar.

Bei einer bevorzugten Ausführungsform des erfindungsgemäßen Verfahrens liegt die Zellaustragsrate bzw. Verdünnungsrate D (= täglich ausgetauschtes Volumen / Arbeitsvolumen des Fermenters) im Bereich von 0,1 bis 12 (=1/10 bis12/1) je nach Wachstumsrate des Ciliatenstammes.
Die Erfindung wird im folgenden anhand von Ausführungsbeispielen näher erläutert.

### Beispiel 1: Kontinuierliche Fermentation von Tetrahymena pyriformis Tetrahymena pyriformis wurde in einem 2 1 Fermenter des Typs Biostat MD (Braun Biotech, Melsungen) unter folgenden Bedingungen kultiviert:

### Medium:

Wasser mit Zusätzen von
- 0,5 Gewichts-% Proteose Pepton
- 0,1 Gewichts-% Hefeextrakt
- 3 Gewichts-% flüssiger Stärkezucker
- 1 ml/l Eisenspur

Fermentationsbedingungen:
- Temperatur: 30°C
- Sauerstoffsättigung: 20 %
- pH-Regulierung: pH 7
- Start (t ₀ₕ): Inokulum 50.000 Zellen / ml; Kultivierung nach Batch-Verfahren-Art
- Beginn der kontinuierlichen Fermentation: t₅₁ₕ mit D = 1;
- Fortsetzung der kontinuierlichen Fermentation: ab t₇₈ₕ mit D = 1,5; ab t₉₈ₕ mit D = 2,4

Zu Beginn der Kultivierung wurde das Medium mit etwa 50.000 Zellen angeimpft und diese Starterkultur nach Art eines Batch-Verfahrens so lange kultiviert, bis die Zellpopulation am Ende der Vermehrungsphase und kurz vor dem Eintritt in die stationäre Phase stand. Zu diesem Zeitpunkt - im vorliegenden Beispiel 51 Stunden nach der Animpfung (t₅₁ₕ) - wurde auf kontinuierliche Fermentation umgestellt, d.h. von da an wurde kontinuierlich zellhaltiges Medium abgenommen und die entsprechende Menge zellfreies Medium zugeführt. Zu Beginn der kontinuierlichen Fermentation betrug die Zellaustrags- bzw. Verdünnungsrate (=Volumenmenge an täglich ausgetauschtem Medium pro Arbeitsvolumen des Fermenters) D=1, d.h. pro Tag wurde der gesamte Inhalt des Fermenters (2 l) einmal ausgetauscht und 2 l Ciliaten-haltiges Medium gewonnen. Dieses Medium enthielt etwa 1 Millionen Zellen pro ml.

27 Stunden nach Beginn der kontinuierlichen Fermentation (= 78 Stunden nach der Animpfung= t₇₈ₕ)wurde die Zellaustragsrate bzw. Verdünnungsrate auf D=1,5 erhöht, d.h. pro Tag wurden ab diesem Zeitpunkt etwa 3 1 Ciliaten-haltiges Medium gewonnen. Die Zelldichte blieb dabei praktisch unverändert bei etwa 1 Millionen Zellen pro ml. Nach weiteren 20 Stunden (= 98 Stunden nach der Animpfung= t₉₈ₕ) wurde die Zellaustragsrate bzw. Verdünnungsrate nochmals erhöht auf D=2,4, d.h. pro Tag wurden etwa 5 l Ciliaten-haltiges Medium gewonnen, wobei die Zelldichte nach wie vor praktisch unverändert bei etwa 1 Millionen Zellen / ml lag.

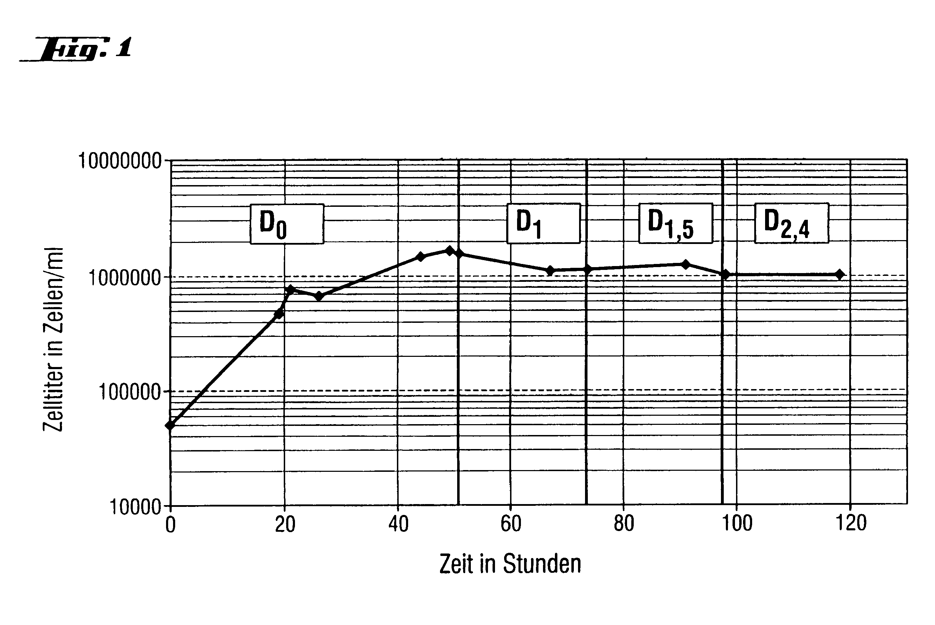

Die Ergebnisse dieses Fermentationsprozesses sind in Fig. 1 graphisch darstellt. Aus dem dort abgebildeten Kurvenverlauf ist ersichtlich, daß es trotz kontinuierlichem Zellaustrag zu keiner Ausverdünnung kam, sondern eine ständige und kontinuierliche Vermehrung der Ciliaten stattfand. Mit anderen Worten: die Kultur befand sich auch bei größerem Zellaustrag (D=2,4) immer in einem dynamischen Gleichgewicht zwischen Zellaustrag und Zellvermehrung.

### Beispiel 2: Kontinuierliche Fermentation von Tetrahymena thermophila Tetrahymena thermophila wurde in einem 2 1 Fermenter des Typs Biostat MD (Braun Biotech, Melsungen) unter folgenden Bedingungen kultiviert:

### Medium:

Wasser mit Zusätzen von
- 5 g/l Proteose Pepton
- 1 g/l Hefeextrakt
- 1 ml/l Eisenspur
- 1 Gewichts-% Glucose in Form von flüssigem Stärkezucker

Fermentationsbedingungen:
- Temperatur: 30°C
- Sauerstoffsättigung: 20 %
- pH-Regulierung: pH 7
- Start (tₒₕ) : Inokulum 50.000 Zellen / ml; Kultivierung nach Batch-Verfahren-Art
- Beginn der kontinuierliche Fermentation: t₄₅ₕ mit D = 1,2;
- Fortsetzung der kontinuierlichen Fermentation: ab t₁₇₈ₕ mit D = 2,4; ab t₁₉₀ₕ mit D = 3

Das Verfahren wurde im Prinzip wie unter Beispiel 1 beschrieben durchgeführt.

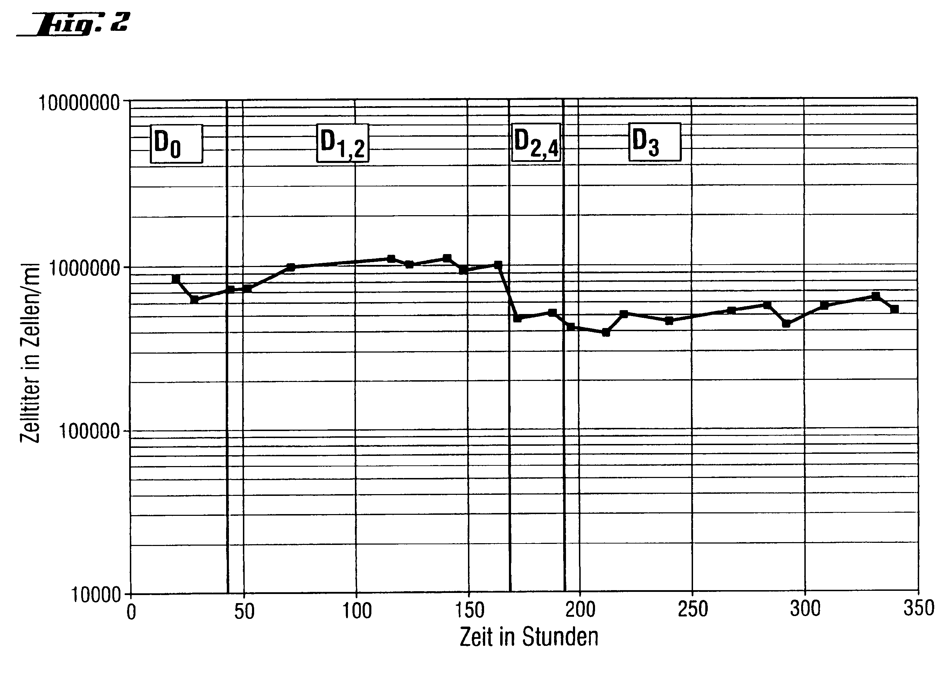

In Fig. 2 ist das Wachstums- bzw. Vermehrungsverhalten der Ciliatenpopulation unter den genannten Fermentationsbedingungen graphisch darstellt. Aus dem abgebildeten Kurvenverlauf ist erkennbar, daß die Steigerung der Zellaustrags- bzw. Verdünnungsrate von D=1,2 auf D=2,4 (Verdoppelung) zu einer Abnahme der Zelldichte von etwa 1 Millionen Zellen/ml auf etwa 500.000 Zellen/ml (Halbierung) führte, daß diese Zelldichte dann aber relativ konstant blieb und nicht weitere abnahm, selbst bei einer weiteren Steigerung der Zellaustrags- bzw. Verdünnungrate von D=2,4 auf D=3.

### Beispiel 3: Kontinuierliche Fermentation von Tetrahymena thermophila

### Medium:

Wasser mit Zusätzen von
- 20 g/l Magermilchpulver
- 10 g/l Glucose
- 5 g/l Hefeextrakt
- 1 ml/l Eisenspur

Ferrnentationsbedingungen:
- Temperatur: 30°C
- Sauerstoffsättigung: 20 %
- Rührer: als 2. Kaskade für Sauerstoffregulation
- pH-Regulierung: pH 7
- Start (t_{Oh}): Inokulum 50.000 Zellen / ml; Kultivierung nach Batch-Verfahren-Art
- Beginn der kontinuierlichen Fermentation: t ₂₀ₕ mit D = 1,125;
- Fortsetzung der kontinuierlichen Fermentation ab t₆₈ₕ mit D = 1,9;
   ab t₁₃₉ₕ mit D = 4,14;
   ab t₁₆₈ₕ mit D = 4,94

Das Verfahren wurde im Prinzip wie unter Beispiel 1 beschrieben durchgeführt.

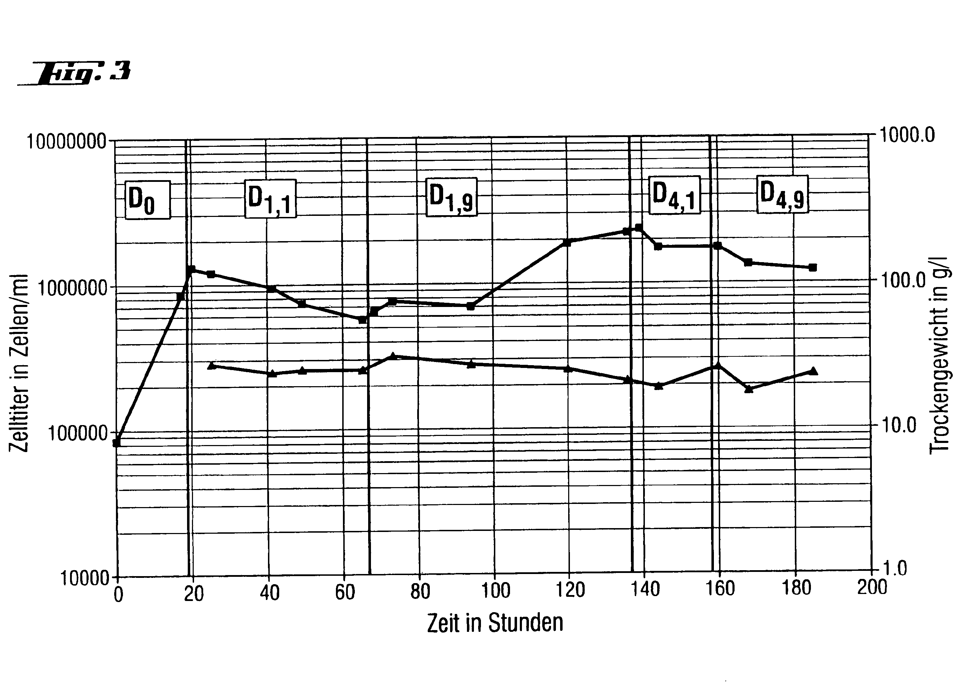

In Fig. 3 ist das Wachstums- bzw. Vermehrungsverhalten der Ciliatenpopulation unter den vorstehend genannten Fermentationsbedingungen graphisch darstellt. Der abgebildete Kurvenverlauf zeigt, daß es zu Beginn der kontinuierlichen Fermentation zu einer Abnahme der Zelldichte von anfänglich etwa 1 Millionen Zellen pro ml auf etwa 600.000 Zellen pro ml kam. Die Zellpopulation erholte sich aber wieder trotz einer Steigerung der Zellaustrags- bzw. Verdünnungrate innerhalb von 1,5 Tagen (etwa 36 Stunden), und hatte etwa 4 Tage (90 Stunden) nach Beginn der kontinuierlichen Fermentation wieder ihre Ausgangsdichte von 1 Millionen Zellen pro ml erreicht. Dieser Wert wurde auch bei weiterer Steigerung der Zellaustrags- bzw. Verdünnungsrate auf D=4,1 und schließlich auf D=4,9 nicht mehr unterschritten.

In der unteren Kurve in Fig. 3 sind die Ergebnisse von Bestimmungen des Trockengewichts der Zellen (in g pro l) während der Kultivierungsdauer dargestellt. Aus dem im wesentlichen parallel zur Zellvermehrungskurve verlaufenden Kurvenverlauf ist erkennbar, daß die Zellvermehrung nicht auf Kosten der Zellgröße bzw. des Zellvolumens der einzelnen Ciliatenzellen erfolgt, sondern daß tatsächlich entsprechend mehr Biomasse produziert wird.

### Beispiel 4: Kontinuierliche Fermentation von Colpidium campylum Colpidium campylum wurde in einem 2-I Fermenter des Typs Biostat MD (Braun Biotech. Melsungen) unter folgenden Bedingungen kultiviert:

### Medium:

Wasser mit Zusätzen von
- 20g/l Magermilchpulver
- 10 g/l Glucose
- 5 g/l Hefeextrakt
- 1 ml/l Eisenspur

Fermentationsbedingungen:
- Temperatur: 25°C
- Sauerstoffsättigung: 20%
- Rührer: 108 upm
- pH-Regulierung: pH 7
- Start (t_{Oh}) : Inokulum 50.000 Zellen / ml; Kultivierung nach Batch-Verfahren-Art
- Beginn der kontinuierlichen Fermentation: t _{114,75h} mit D = 0,665;
- Fortsetzung der kontinuierlichen Fermentation ab t ₁₄₀ₕ mit D = 0,632
   ab t ₁₅₉ₕ mit D = 0,462

Das Verfahren wurde im Prinzip wie unter Beispiel 1 beschrieben durchgeführt.

## Patentansprüche

1. Fermentationsverfahren mit kontinuierlicher Massenkultivierung von Ciliaten (Protozoa) zur Produktion biogener Wertstoffe, bei dem die die biogenen Wertstoffe enthaltende Biomasse durch kontinuierlichen Zellaustrag gewonnen wird, **dadurch gekennzeichnet, daß** die Ciliatenzellen in komplexem axenischem Medium kultiviert werden.

2. Fermentationsverfahren nach Anspruch 1, **dadurch gekennzeichnet, daß** die Ciliaten einer der taxonomischen Gruppen Holotricha, Peritricha, Spirotricha und Suctoria, insbesondere die Gattungen Tetrahymena, Paramecium, Colpoda, Glaucoma, Parauronema, Engelmanniella, Stylonichia, Euplotes und Colpidium, angehören, wobei neben den Wildstämmen auch Mutanten und/oder Rekombinanten dieser Stämme umfaßt sind.

3. Fermentationsverfahren nach Anspruch 1 oder 2, **dadurch gekennzeichnet, daß** die Fermentation in einem Rühr- oder Blasensäulen- oder Airliftfermenter durchgeführt wird.

4. Fermentationsverfahren nach einem der Ansprüche 1 bis 3, **dadurch gekennzeichnet, daß** die Fermentation bei einem pH-Wert im Bereich von pH 4 bis pH 9 und/oder einer Fermentationstemperatur im Bereich von etwa 15 bis etwa 40°C durchgeführt wird.

5. Fermentationsverfahren nach einem der Ansprüche 1 bis 4, **dadurch gekennzeichnet, daß** das Medium eine Kohlenstoff-Quelle enthält, die eine oder mehrere Substanzen aus der Gruppe: Glucose, Fructose, Xylose, Saccharose, Maltose, Stärke, Fucose, Glucosamin, Lactose, Melasse, Dextran, Fettsäuren (z.B. Ölsäure), Sojaöl. Sonnenblumenöl, Glycerin, Glutaminsäure, Mannitol. Mager-milchpulver und Acetat umfaßt.

6. Fermentationsverfahren nach Anspruch 5, **dadurch gekennzeichnet, daß** die Konzentration der Kohlenstoff-Quelle einen Wert im Bereich von etwa 0,2 bis etwa 20 Gewichts-%, bezogen auf das Kulturmedium, beträgt.

7. Fermentationsverfahren nach einem der Ansprüche 1 bis 6, **dadurch gekennzeichnet, daß** das Medium eine Stickstoff-Quelle enthält, die eine oder mehrere Substanzen aus der Gruppe: Peptone, Hefeextrakt, Malzextrakt, Fleischextrakt, Magermilchpulver, Casamino Acid, Corn Steep Liquor, Na-Glutamat, Harnstoff, Ammoniumacetat, Ammoniumsulfat, Ammoniumchlorid und Ammoniumnitrat umfaßt.

8. Fermentationsverfahren nach Anspruch 7, **dadurch gekennzeichnet, daß** die Konzentration der Stickstoff-Quelle einen Wert im Bereich von etwa 0,1 bis etwa 10 Gewichts-%, bezogen auf das Kulturmedium, beträgt.

9. Fermentationsverfahren nach einem der Ansprüche 1 bis 8, **dadurch gekennzeichnet, daß** das Medium wenigstens eine Phosphatquelle enthält, vorzugsweise Kaliumphosphat und/oder Kalium-Dihydrogenphosphat.

10. Fermentationsverfahren nach einem der Ansprüche 1 bis 9, **dadurch gekennzeichnet, daß** das Medium eine oder mehrere der folgenden Substanzen enthält: Ammoniumsulfat, Natriumsulfat , Magnesium, Eisen, Kupfer, Calcium, Vitamine, Spurenelemente.

11. Fermentationsverfahren nach einem der Ansprüche 1 bis 10, **dadurch gekennzeichnet, daß** das Medium abgetötete Biomasse von Futterorganismen der Ciliaten enthält.

12. Fermentationsverfahren nach einem der Ansprüche 1 bis 11, **dadurch gekennzeichnet, daß** die im Zellaustrag enthaltenen Zellen (= geerntete Biomasse) mittels Zentrifugation und/oder Tangentialfiltration und/oder Mikrofiltration und/oder Sedimentation und/oder Flotation vom Kulturmedium abgetrennt wird.

13. Fermentationsverfahren nach einem der Ansprüche 1 bis 12, **dadurch gekennzeichnet, daß** die Zellaustragsrate bzw. Verdünnungsrate ( = täglich ausgetauschtes Volumens / Arbeitsvolumen des Fermenters) einen Wert im Bereich von 0,1 bis 12 beträgt.

14. Fermentationsverfahren nach einem der Ansprüche 1 bis 13, **dadurch gekennzeichnet, daß** die biogenen Wertstoffe eine oder mehrere Substanz(en) aus der Gruppe, bestehend aus: Peptiden und Proteine, insbesondere Enzymen, Fettsäuren und Lipide, Polysaccharide, Nukleinsäuren, Sekundärmetabolite und Polymere, sind oder aber die Biomasse selbst einen Wertstoff (z.B. Tierfutter) darstellt.

## Claims

1. A fermentation process with continuous mass cultivation of ciliates (protozoa) for producing biogenous valuable substances, where the biomass containing the biogenous valuable substances is obtained by continuous cell extraction, which comprises cultivating the ciliate cells in a complex axenic medium.

2. The fermentation process as claimed in claim 1, wherein the ciliates belong to one of the taxonomic groups Holotricha, Peritricha, Spirotricha and Suctoria, in particular to the orders Tetrahymena, Paramecium, Colpoda, Glaucoma, Parauronema, Engelmanniella, Stylonichia, Euplotes and Colpidium, which include, in addition to the wild strains, also mutants and/or recombinants of these strains.

3. The fermentation process as claimed in claim 1 or 2, wherein the fermentation is carried out in a stirred or bubble column or airlift fermenter.

4. The fermentation process as claimed in any of claims 1 to 3, wherein the fermentation is carried out at a pH in the range from pH 4 to pH 9 and/or a fermentation temperature in the range from about 15 to about 40°C.

5. The fermentation process as claimed in any of claims 1 to 4, wherein the medium contains a carbon source which comprises one or more substances from the group consisting of: glucose, fructose, xylose, sucrose, maltose, starch, fucose, glucosamine, lactose, molasses, dextran, fatty acids (for example oleic acid), soya oil, sunflower oil, glycerol, glutamic acid, mannitol, skim-milk powder and acetate.

6. The fermentation process as claimed in claim 5, wherein the concentration of the carbon source has a value in the range from about 0.2 to about 20% by weight, based on the culture medium.

7. The fermentation process as claimed in any of claims 1 to 6, wherein the medium contains a nitrogen source which comprises one or more substances from the group consisting of: peptones, yeast extract, malt extract, meat extract, skim-milk powder, casamino acid, corn steep liquor, Na-glutamate, urea, ammonium acetate, ammonium sulfate, ammonium chloride and ammonium nitrate.

8. The fermentation process as claimed in claim 7, wherein the concentration of the nitrogen source has a value in the range from about 0,1 to about 10% by weight, based on the culture medium.

9. The fermentation process as claimed in any of claims 1 to 8, wherein the medium contains at least one phosphate source, preferably potassium phosphate and/or potassium dihydrogen phosphate.

10. The fermentation process as claimed in any of claims 1 to 9, wherein the medium contains one or more of the following substances: ammonium sulfate, sodium sulfate, magnesium, iron, copper, calcium, vitamins, trace elements.

11. The fermentation process as claimed in any of claims 1 to 10, wherein the medium contains killed biomass of feed organisms of the ciliates.

12. The fermentation process as claimed in any of claims 1 to 11, wherein the cells contained in the cell extract (= harvested biomass) are separated off from the culture medium by centrifugation and/or tangential filtration and/or microfiltration and/or sedimentation and/or flotation.

13. The fermentation process as claimed in any of claims 1 to 12, wherein the cell extraction rate or dilution rate (= the volume that is exchanged per day/the operating volume of the fermenter) has a value in the range from 0.1 to 12.

14. The fermentation process as claimed in any of claims 1 to 13, wherein the biogenous valuable substances are one or more substance(s) from the group consisting of: peptides and proteins, in particular enzymes, fatty acids and lipids, polysaccharides, nucleic acids, secondary metabolites and polymers, or else the biomass itself is a valuable substance (for example animal feed).

## Revendications

1. Procédé de fermentation avec la culture continue en masse des cillés (protozoaires) pour la production de substances biogènes utiles, où la biomasse qui contient les substances blogèhes utiles est extraite par l'excrétion continue des cellules, **caractérisé en ce que**, les cellules des ciliées sont cultivées dans un milieu complexe, axénique.

2. Procédé de fermentation selon la revendication 1, **caractérisé en ce que**, les ciliés appartiennent à l'une d'entre les groupes taxonomiques Holotricha, Peritricha, Spirotricha et Suctoria, particulièrement à l'une d'entre les espèces Tetrahymena, Paramecium, Colpoda, Glaucoma, Parauronema, Engelmannlella, Stylonichia, Euplotes et Colpidium, où à côté des tiges sauvages sont inclus aussi des mutants ou recombinants de ces tiges.

3. Procédé de fermentation selon la revendication 1 ou 2, **caractérisé en ce que**, la fermentation se réalise dans une cuve de fermentation à agitateur, à bulles ou aérateur.

4. Procédé de fermentation selon l'une des revendications 1 à 3, **caractérisé en ce que**, la fermentation se réalise à une valeur pH dans l'intervalle pH 4 à pH 9 et/ou à une température de fermentation dans l'intervalle d'environ 15 à environ 40° C.

5. Procédé de fermentation selon l'une des revendications 1 à 4, **caractérisé en ce que**, le milieu contient une source de carbone, qui comprend l'une ou plusieurs substances des groupes: glucose, fructose, xylose, saccharose, maltose, amidon, fucose, aminoglucose, lactose, mélasse, dextran, acides grasses (par exemple, acide oléique), huile de soya, huile de tournesol, glycérine, acide glutamique, sucre de manne, lait en poudre dégraissé, et acétate.

6. Procédé de fermentation selon la revendication 5, **caractérisé en ce que**, la concentration de la source de carbone présente des valeurs dans l'intervalle d'environ 0,2 à environ 20 pour cents du poids, rapportée au milieu de culture.

7. Procédé de fermentation selon l'une des revendications 1 à 6, **caractérisé en ce que**, le milieu contient une source de nitrogène, qui comprend l'une ou plusieurs substances des groupes:Peptone, extrait de levure, extrait de malt, extrait de viande, lait en poudre dégraissé, acide Casamino, Corn Steep Liquor, glutamate de Na, urée, acétate d'ammonium, sulfate d'ammonium, chlorure d'ammonium et nitrate d'ammonium.

8. Procédé de fermentation selon la revendication 7, **caractérisé en ce que**, la concentration de la source de nitrogène présente des valeurs dans l'intervalle d'environ 0,1 à environ 10 pour cents du poids, rapportée au milieu de culture.

9. Procédé de fermentation selon l'une des revendications 1 à 8, **caractérisé en ce que**, le milieu contient au moins une source de phosphate, de préférence phosphate de potassium et/ou dihydrogène phosphate de potassium.

10. Procédé de fermentation selon l'une des revendications 1 à 9, **caractérisé en ce que**, le milieu contient l'une ou plusieurs des suivantes substances: sulfate d'ammonium, sulfate de sodium, magnésium, fer, cuivre, calcium, vitamines, oligo-éléments,

11. Procédé de fermentation selon l'une des revendications 1 à 10, **caractérisé en ce que**, le milieu contient biomasse morte d'organismes fourragers des ciliés.

12. Procédé de fermentation selon l'une des revendications 1 à 11, **caractérisé en ce que**, les cellules contenues dans l'excrétion de cellules (=biomasse récoltée) sont séparées par centrifugation et/ou par filtrage tangentiel et/ou par microfiltrage et/ou par sédimentation et/ou par flottation du milieu de culture.

13. Procédé de fermentation selon l'une des revendications 1 à 12, **caractérisé en ce que**, l'indice d'excrétion des cellules respectivement l'indice de raréfaction (= volume journalier remplacé / volume de travail de la cuve de fermentation) présente des valeurs dans l'intervalle de 0,1 à 12.

14. Procédé de fermentation selon l'une des revendications 1 à 13, **caractérisé en ce que**, les substances biogènes utiles sont l'une ou plusieurs substances des groupes constitués de: peptide et protéine, particulièrement enzymes, acides grasses et lipides, polysaccharides, acides nucléiques, métabolites secondaires et polymères, ou la biomasse représente elle-même une substance utile (par exemple fourrage).
